# EUROPÄISCHE PATENTSCHRIFT

(11) **EP 3 949 906 B1**
(45) Veröffentlichungstag und Bekanntmachung des Hinweises auf die Patenterteilung: **07.05.2025**
(21) Anmeldenummer: 21189595.8
(22) Anmeldetag: 04.08.2021
(51) Int. Cl.: A61F 2/32, A61F 2/34, A61F 2/36, A61F 2/30

(54) **KUGELGELENKENDOPROTHESE**
BALL JOINT ENDOPROSTHESIS
ENDOPROTHÈSE D'ARTICULATION À ROTULE

(30) Priorität: 07.08.2020 DE 102020210033
(43) Veröffentlichungstag der Anmeldung: 09.02.2022
(73) Patentinhaber: Aesculap AG, 78532 Tuttlingen (DE)
(72) Erfinder: Hirt, Martin, 78333 Stockach (DE); Reu, Gerhard, 78532 Tuttlingen (DE)
(74) Vertreter: Patentanwälte Ruff, Wilhelm, Beier, Dauster & Partner mbB

(56) Entgegenhaltungen:
- WO-A1-2013/148434
- WO-A2-2007/069250
- FR-B1- 2 795 302
- US-A1- 2003 171 817
- US-A1- 2012 203 351
- US-A1- 2015 148 910

## Beschreibung

Die Erfindung betrifft eine Kugelgelenkendoprothese, insbesondere Hüftendoprothese, mit einem Kugelgelenkkopf, der eine konvexe Artikulationsfläche aufweist, und mit einer Kugelgelenkpfanne, die eine konkave Artikulationsfläche aufweist, wobei die konvexe Artikulationsfläche und die konkave Artikulationsfläche kugelgelenkig artikulierend gleitbeweglich zusammenwirken.

Eine derartige Kugelgelenkendoprothese ist in Form einer Hüftendoprothese aus der US 2008/0228282 A1 bekannt und weist einen Kugelgelenkkopf und eine Kugelgelenkpfanne auf. Der Kugelgelenkkopf weist eine konvexe Artikulationsfläche auf. Die Kugelgelenkpfanne weist eine konkave Artikulationsfläche auf. Die beiden Artikulationsflächen wirken kugelgelenkig artikulierend gleitbeweglich zusammen. Bei der bekannten Kugelgelenkendoprothese weist der Kugelgelenkkopf Einkerbungen auf. Diese Einkerbungen sind als schmale nutförmige Einschnitte in die konvexe Artikulationsfläche eingebracht und sollen einer Reibungsminderung dienen. Die US 2003/171817 A1 offenbart die technischen Merkmale der Präambel von Anspruch 1.

Aufgabe der Erfindung ist es, eine Kugelgelenkendoprothese der eingangs genannten Art bereitzustellen, die gegenüber dem Stand der Technik verbesserte Verschleißeigenschaften aufweist.

Diese Aufgabe wird dadurch gelöst, dass der Kugelgelenkkopf mehrere benachbart angeordnete und radial abgeflachte Abflachungsflächen aufweist, wodurch die konvexe Artikulationsfläche in Form einer modifizierten Kugelfläche ausgebildet ist, die - im Vergleich zu einer gedachten nicht-modifizierten Kugelfläche - eine um die Abflachungsflächen reduzierte Oberfläche zur Artikulation mit der konkaven Artikulationsfläche aufweist. Dabei geht die Erfindung von der Überlegung aus, dass ein reibungsbedingter Abrieb zwischen dem Kugelgelenkkopf und der Kugelgelenkpfanne unter anderem von der Größe, d. h. Oberfläche, der bei der Artikulation wirksamen Kontaktfläche und dem dabei vorherrschenden Reibungskoeffizienten abhängig ist. Durch die erfindungsgemäße Lösung wird die wirksame Kontaktfläche des Kugelgelenkkopfs in ihrer Größe, d. h. Oberfläche, reduziert. Hierzu ist der Kugelgelenkkopf mit den besagten Abflachungsflächen versehen. Im Bereich der Abflachungsflächen liegt kein Kontakt zwischen dem Kugelgelenkkopf und der Kugelgelenkpfanne vor. Mit anderen Worten ausgedrückt, ist die wirksame Kontaktfläche zwischen dem Kugelgelenkkopf und der Kugelgelenkpfanne - im Vergleich zu einer Gestaltung ohne Abflachungsflächen - um die Abflachungsflächen reduziert. Hierdurch können auf überraschend einfache und wirkungsvolle Weise der reibungsbedingte Abrieb reduziert und verbesserte Verschleißeigenschaften der Kugelgelenkendoprothese erreicht werden. Hinsichtlich der Verschleißeigenschaften wirkt sich die radial abgeflachte Gestalt der Abflachungsflächen zusätzlich vorteilhaft aus. Denn im Unterschied zu den aus dem Stand der Technik bekannten nutförmigen Einschnitten bilden die Abflachungsflächen keine scharfkantigen Rücksprünge. Stattdessen gehen die Abflachungsflächen sprungfrei und/oder ohne scharfe Kanten in benachbarte Flächenabschnitte der konvexen Artikulationsfläche des Kugelgelenkkopfs über. Die Erfinder haben erkannt, dass dieser sprungfreie und/oder nichtscharfkantige Übergang vergleichsweise abriebmindernd wirkt. Hierdurch kann im Vergleich zu den besagten nutförmigen Einschnitten mittels der Abflachungsflächen ein verbessertes Verschleißverhalten erreicht werden. "Sprungfrei" meint insbesondere, dass die Abflachungsflächen in radialer Richtung relativ zu der konvexen Artikulationsfläche nicht nach innen und/oder außen versetzt sind. Mit anderen Worten ausgedrückt, gehen die Abflachungsflächen stufenlos und/oder ohne Versatz in die konvexe Artikulationsfläche über und umgekehrt. Ein solcher Sprung, Versatz bzw. eine solche Stufe liegt insbesondere bei einer Kerbe, Nut, Einbuchtung und/oder einem Einschnitt vor, nicht jedoch bei den Abflachungsflächen. Der Kugelgelenkkopf weist entlang seiner Längengrade und/oder Breitengrade eine stets konvexe Krümmung auf, wobei die Krümmung im Bereich der Abflachungsflächen einen größeren Krümmungsradius aufweist als im Bereich der konvexen Artikulationsfläche. Bei einer Ausgestaltung geht der Krümmungsradius im Bereich der Abflachungsflächen gegen unendlich, d.h. die Abflachungsflächen sind in einer Erstreckungsrichtung plan. Zur Ausbildung der Abflachungsflächen wird der Kugelgelenkkopf bzw. dessen Kugelfläche abschnittsweise radial abgeflacht. Fertigungstechnisch kann dies abrasiv oder nicht-abrasiv erfolgen. Beispielsweise kann der Kugelgelenkkopf abschnittsweise an mehreren benachbarten Stellen plan geschliffen werden. In diesem Fall bilden die plan geschliffenen Stellen oder Abschnitte die besagten Abflachungsflächen. Die Abflachungsflächen sind jeweils in einer ersten Erstreckungsrichtung plan, eben und/oder nichtkonkav erstreckt und in einer senkrecht zur ersten Erstreckungsrichtung orientierten zweiten Erstreckungsrichtung gemäß der Kugelform des Kugelgelenkkopfs gekrümmt erstreckt. Die mehreren Abflachungsflächen sind benachbart zueinander angeordnet und können hierzu in Umfangsrichtung und/oder in Polrichtung des Kugelgelenkkopfs voneinander, insbesondere gleichmäßig, beabstandet sein. Vorzugsweise sind die mehreren Abflachungsflächen gleichförmig gestaltet und/oder hinsichtlich ihrer Erstreckung gleichgerichtet auf dem Kugelgelenkkopf orientiert. Die mehreren Abflachungsflächen können grundsätzlich eine beliebige Flächenform aufweisen. Vorzugsweise sind die Abflachungsflächen jeweils streifenförmig durchgängig längserstreckt und bilden auf diese Weise jeweils einen Kapillarspalt zwischen dem Kugelgelenkkopf und der Kugelgelenkpfanne aus. Eine solche Gestaltung ist besonders vorteilhaft, aber nicht als zwingend erforderlich und/oder wesentlich im Hinblick auf die vorliegende Erfindung zu erachten. Die Kugelgelenkendoprothese ist vorzugsweise eine Hüftendoprothese. Alternativ kann die Kugelgelenkendoprothese eine Schultergelenkendoprothese sein. Vorzugsweise weist der Kugelgelenkkopf keine, insbesondere zur Reibungsminderung eingerichtete, Nuten, Einschnitte, Einsenkungen und/oder Kerben auf.

Die modifizierte Kugelfäche weist im Vergleich zu der gedachten nicht-modifizierten Kugelfläche eine um 25 % bis 50 %, bevorzugt um 40 %, reduzierte Oberfläche zur Artikulation mit der konkaven Artikulationsfläche auf. Die Erfinder haben zum einen erkannt, dass eine Oberflächenreduktion von weniger als 25 % zu klein ist, um eine signifikante Verringerung des Abriebs zu ermöglichen. Zum anderen haben die Erfinder erkannt, dass eine Oberflächenreduktion von mehr als 50 % die zur Artikulation wirksame Kontaktfläche zwischen dem Kugelgelenkkopf und der Kugelgelenkpfanne, die auch als Tragfläche bezeichnet werden kann, zu stark verringert. Dies kann insbesondere zu einer erhöhten Flächenpressung führen, die der angestrebten Verschleißreduktion abträglich sein kann. Insoweit hat sich der vorgenannte Wertebereich zwischen 25 % und 50 % als vorteilhaft erwiesen. Eine Oberflächenreduktion um 40 % stellt einen besonders vorteilhaften Kompromiss zwischen signifikanter Reibungsreduzierung einerseits und andererseits ausreichender Tragfähigkeit dar.

In weiterer Ausgestaltung der Erfindung sind die Abflachungsflächen in Umfangsrichtung des Kugelgelenkkopfs gleichmäßig beabstandet angeordnet. Eine solche Anordnung bietet besondere Vorteile. Gleichmäßig beabstandet meint, dass die Abflachungsflächen mit einem konstanten Winkelversatz in Umfangsrichtung des Kugelgelenkkopfs angeordnet sind. Dabei ergibt sich der konstante Winkelversatz als Quotient aus 360° geteilt durch n, wobei n die Anzahl der Abflachungsflächen repräsentiert.

Der Kugelgelenkkopf ist zum Ausbilden der Abflachungsflächen abschnittsweise plan geschliffen. Alternativ kann der Kugelgelenkkopf zum Ausbilden der Abflachungsflächen nicht-abrasiv bearbeitet werden. Denkbar ist insbesondere ein Umformen des Kugelgelenkkopfs. Bei einer Ausgestaltung ist wenigstens eine der Abflachungsflächen mittels Planschleifens ausgebildet. Bei weiteren Ausgestaltungen sind mehr als eine, vorzugsweise sämtliche, Abflachungsflächen mittels Planschleifens ausgebildet.

Die Abflachungsflächen sind jeweils in einer ersten Erstreckungsrichtung plan erstreckt und in einer senkrecht zu der ersten Erstreckungsrichtung orientierten zweiten Erstreckungsrichtung gekrümmt erstreckt. Vorzugsweise ist die erste Erstreckungsrichtung parallel zu einer Umfangsrichtung des Kugelgelenkkopfs. Vorzugsweise ist die zweite Erstreckungsrichtung eine Haupt- und/oder Längserstreckungsrichtung der jeweiligen Abflachungsfläche.

In weiterer Ausgestaltung der Erfindung sind die Abflachungsflächen jeweils ausgehend von einem unterseitigen Schaftbereich des Kugelgelenkkopfs bis in einen oberseitigen Polbereich des Kugelgelenkkopfs durchgängig längserstreckt, wodurch jeweils ein durchgängig längserstreckter Kapillarspalt zum kapillaren Transport von Gelenkflüssigkeit ausgebildet ist. Dies ist eine besonders vorteilhafte Ausgestaltung der Erfindung. Denn durch die durchgängige Längserstreckung der Abflachungsflächen sind mehrere dementsprechend durchgängig längserstreckte Kapillarspalte zwischen der Kugelgelenkpfanne und dem Kugelgelenkkopf ausgebildet. Diese Kapillarspalte sind zum kapillaren Transport von Gelenkflüssigkeit vorgesehen. Die Gelenkflüssigkeit kann ausgehend von dem Schaftbereich des Kugelgelenkkopfs entlang der Kapillarspalte bis in den Polbereich des Kugelgelenkkopfs gelangen. Hierdurch wird eine verbesserte Schmierung der gleitbeweglichen Artikulation zwischen dem Kugelgelenkkopf und der Kugelgelenkpfanne erreicht. Dies führt zu einem verminderten Abrieb und letztlich zu einem nochmals verbesserten Verschleißverhalten der Kugelgelenkendoprothese. Der Schaftbereich des Kugelgelenkkopfs ist, sofern die Kugelgelenkendoprothese eine Hüftendoprothese ist, femurseitig orientiert. Der Polbereich ist dem Schaftbereich abgewandt und kann insbesondere hüftseitig orientiert sein. Durch die durchgängige Längserstreckung zwischen dem Schaft- und dem Polbereich weisen die Abflachungsflächen eine streifenförmige, bahnförmige oder anderweitig längliche Gestalt auf.

In weiterer Ausgestaltung der Erfindung sind die Abflachungsflächen jeweils um 0,03 mm bis 0,3 mm, bevorzugt um 0,1 mm, gegenüber der gedachten nicht-modifizierten Kugelfläche abgeflacht. Diese Ausgestaltung der Erfindung ist insbesondere, aber nicht ausschließlich, im Hinblick auf den vorgenannten Kapillareffekt besonders vorteilhaft. Denn durch die Abflachung der Abflachungsflächen in dem genannten Wertebereich kann eine ausreichende kapillare Steighöhe der Gelenkflüssigkeit innerhalb der Kapillarspalte erreicht werden. Dies gewährleistet, dass die Gelenkflüssigkeit im Wesentlichen die vollständige konvexe Artikulationsfläche bzw. modifizierte Kugelfläche des Kugelgelenkkopfs benetzen kann. Sofern die Abflachungsflächen gemäß der vorhergehenden Ausgestaltung durchgängig längserstreckt und entsprechende Kapillarspalte ausgebildet sind, weisen diese dementsprechend ein Spaltmaß zwischen 0,03 mm und 0,3 mm, bevorzugt 0,1 mm, auf. Die Abflachungsflächen können jeweils mit einer entlang ihrer Längserstreckung veränderlichen oder konstanten Abflachung ausgebildet sein.

In weiterer Ausgestaltung der Erfindung sind die Abflachungsflächen in Polrichtung des Kugelgelenkkopfs jeweils abnehmend abgeflacht und gehen im Polbereich des Kugelgelenkkopfs stetig in die modifizierte Kugelfläche über. Dementsprechend weisen die Abflachungsflächen bei dieser Ausgestaltung der Erfindung entlang ihrer Haupterstreckungsrichtung eine veränderliche Abflachung bzw. Abflachungstiefe auf, die in Richtung des Polbereichs abnimmt und letztlich gegen null geht. Dies kann besondere Vorteile bieten. Insbesondere kann eine Beeinträchtigung der Tragfähigkeit des Polbereichs vermieden werden. Denn dieser ist bei dieser Ausgestaltung der Erfindung nicht mit Abflachungsflächen versehen, da diese dort stetig in die modifizierte Kugelfläche übergehen. Hierdurch ist eine nicht reduzierte und insoweit maximale konvexe Artikulationsfläche im Polbereich des Kugelgelenkkopfs gewährleistet. Dies kann insbesondere im Hinblick auf eine statische Belastung der Kugelgelenkendoprothese Vorteile bieten.

In weiterer Ausgestaltung der Erfindung sind die Abflachungsflächen in Bezug auf die Polrichtung des Kugelgelenkkopfs jeweils gerade oder winklig oder wendelförmig längserstreckt. Eine gerade Längserstreckung der Abflachungsflächen ist fertigungstechnisch besonders einfach herstellbar.

In weiterer Ausgestaltung der Erfindung sind zwischen zwei und 20, bevorzugt zwölf, Abflachungsflächen vorgesehen. Die Anzahl der Abflachungsflächen kann insbesondere auf einen Durchmesser des Kugelgelenkkopfs und/oder einen Werkstoff des Kugelgelenkkopfs und/oder eine Werkstoffpaarung zwischen dem Kugelgelenkkopf und der Kugelgelenkpfanne abgestimmt sein.

Die Erfindung betrifft zudem einen Kugelgelenkkopf für eine Kugelgelenkendoprothese gemäß der vorhergehenden Beschreibung, mit einer konvexen Artikulationsfläche, die zur kugelgelenkigen gleitbeweglichen Artikulation mit einer konkaven Artikulationsfläche einer Kugelgelenkpfanne der Kugelgelenkendoprothese vorgesehen ist. Erfindungsgemäß weist der Kugelgelenkkopf mehrere benachbart angeordnete und radial abgeflachte Abflachungsflächen auf, wodurch die konvexe Artikulationsfläche in Form einer modifizierten Kugelfläche ausgebildet ist, die - im Vergleich zu einer gedachten nicht-modifizierten Kugeloberfläche - eine um die Abflachungsflächen reduzierte Oberfläche zur Artikulation mit der konkaven Artikulationsfläche aufweist. Zu mit der erfindungsgemäßen Gestaltung des Kugelgelenkkopfs einhergehenden Vorteilen und erfindungsgemäßen Ausgestaltungen wird auf die Beschreibung der erfindungsgemäßen Kugelgelenkendoprothese und deren Ausgestaltungen verwiesen. Das zu dem dortigen Kugelgelenkkopf Gesagte gilt sinngemäß entsprechend für den erfindungsgemäßen Kugelgelenkkopf und seine Ausgestaltungen.

Die Erfindung ist durch Anspruch 1 definiert.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Perspektivdarstellung eine Ausführungsform einer erfindungsgemäßen Kugelgelenkendoprothese in Form einer Hüftendoprothese,
- Fig. 2: die Kugelgelenkendoprothese nach Fig. 1 in einer schematischen Längsschnittdarstellung,
- Fig. 3: in schematischer Perspektivdarstellung ein nicht gemäß der Erfindung gestalteter Kugelgelenkkopf nach dem Stand der Technik für eine Kugelgelenkendoprothese,
- Fig. 4, 5: in unterschiedlichen schematischen Perspektivdarstellungen eine Ausführungsform eines erfindungsgemäßen Kugelgelenkkopfs der Kugelgelenkendoprothese nach den Fig. 1 und 2 und
- Fig. 6: eine schematische Querschnittsdarstellung des Kugelgelenkkopfs nach den Fig. 4 und 5 entlang einer äquatorialen Schnittebene.

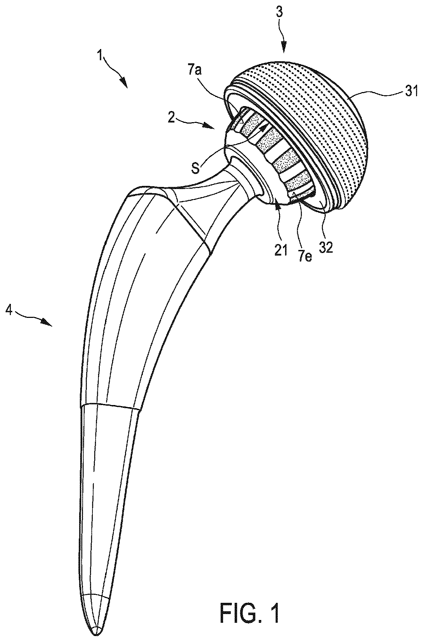

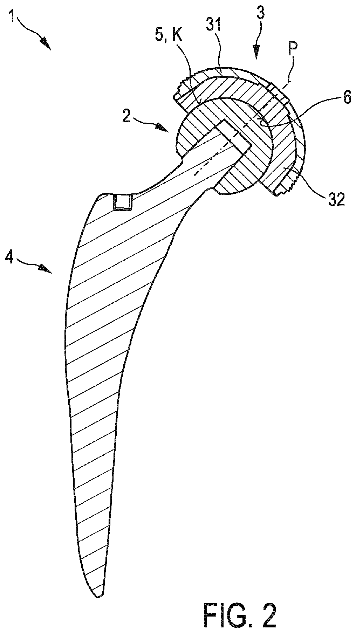

Gemäß den Fig. 1 und 2 ist eine Kugelgelenkendoprothese 1 als künstlicher Gelenkersatz für ein menschliches Kugelgelenk vorgesehen und weist einen Kugelgelenkkopf 2 und eine Kugelgelenkpfanne 3 auf.

Bei der gezeigten Ausführungsform ist die Kugelgelenkendoprothese 1 in Form einer Hüftendoprothese gestaltet, die auch als Hüftimplantat bezeichnet werden kann. Der Kugelgelenkkopf 2 kann insoweit auch als künstlicher Hüftkopf bezeichnet werden. Die Kugelgelenkpfanne 3 kann dementsprechend als künstliche Hüftpfanne bezeichnet werden.

Mit Ausnahme der nachfolgend noch näher beschriebenen erfindungsgemäßen Gestaltung des Kugelgelenkkopfs 2 weist die Kugelgelenkendoprothese 1 eine dem Fachmann grundsätzlich bekannte räumlich-körperliche Gestaltung und Funktionsweise auf, so dass auf eine ausführliche diesbezügliche Beschreibung verzichtet werden kann. Kurz gefasst ist festzuhalten, dass die Kugelgelenkpfanne 3 zur beckenseitigen Fixierung vorgesehen ist. Der Kugelgelenkkopf 2 ist dementgegen zur femurseitigen Fixierung vorgesehen. Zu diesem Zweck ist der Kugelgelenkkopf 2 einends auf eine dem Fachmann bekannte Weise an einem Kugelgelenkschaft 4 angeordnet, der auch als künstlicher Hüftschaft bezeichnet werden kann. Der Kugelgelenkschaft 4 wird auf bekannte Weise in den Femurschaft des Patienten eingesetzt.

Bei der gezeigten Ausführungsform ist die Kugelgelenkpfanne 3 mehrteilig gestaltet und weist ein Schalenteil 31, das auch als Hüftpfannenschale bezeichnet werden kann, und ein Inlayteil 32, das auch als Hüftpfanneneinsatz bezeichnet werden kann, auf. Eine solche mehrteilige Gestaltung der Kugelgelenkpfanne 3 ist im Hinblick auf die Erfindung als nicht wesentlich zu erachten.

Der Kugelgelenkkopf 2 weist eine noch näher beschriebene konvexe Artikulationsfläche 5 auf, die kugelgelenkig artikulierend gleitbeweglich mit einer konkaven Artikulationsfläche 6 der Kugelgelenkpfanne 3 zusammenwirkt (Fig. 2).

Die gleitbewegliche Artikulation zwischen dem Kugelgelenkkopf 2 und der Kugelgelenkpfanne 3, genauer: zwischen der konvexen Artikulationsfläche 5 und der konkaven Artikulationsfläche 6, ist naturgemäß reibungsbehaftet. Dementsprechend lassen sich ein Abrieb und damit einhergehend Verschleißerscheinungen nicht vollumfänglich vermeiden.

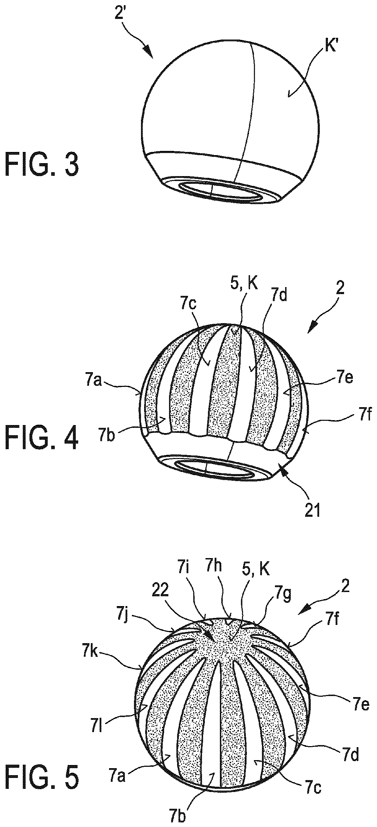

Um dem entgegenzuwirken, weist der Kugelgelenkkopf 2 mehrere benachbart angeordnete und radial abgeflachte Abflachungsflächen 7a bis 7l auf (Fig. 4, 5, 6). Hierdurch ist die konvexe Artikulationsfläche 5 des Kugelgelenkkopfs 2 in Form einer modifizierten Kugelfläche K ausgebildet. Die modifizierte Kugelfläche K weist eine um die Abflachungsflächen 7a bis 7l reduzierte Oberfläche zur Artikulation mit der konkaven Artikulationsfläche 6 der Kugelgelenkpfanne 3 auf. Dabei ist die modifizierte Kugelfläche K kleiner als eine nichtmodifizierte Kugelfläche K' eines mit Ausnahme der Abflachungsflächen 7a bis 7l identisch gestalteten Kugelgelenkkopfs 2', wie er aus dem Stand der Technik bekannt und exemplarisch anhand Fig. 3 gezeigt ist.

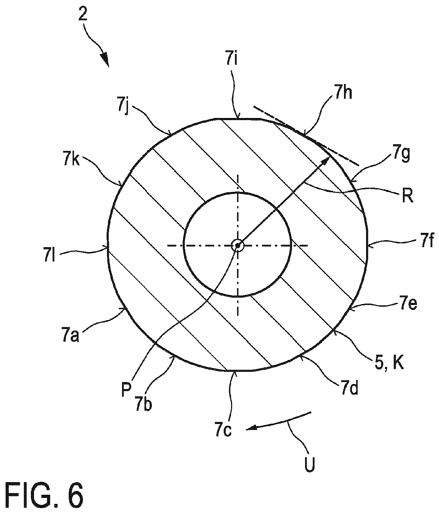

Wie insbesondere anhand Fig. 6 ersichtlich ist, bilden die Abflachungsflächen 7a bis 7l nicht etwa Nuten, Einschnitte oder anderweitige konkave Einkerbungen an dem Kugelgelenkkopf 2 aus. Stattdessen sind die Abflachungsflächen 7a bis 7l plan, eben und/oder nicht konkav. Dies jedenfalls in Bezug auf eine Umfangsrichtung des Kugelgelenkkops 2. Hierdurch werden Rücksprünge und/oder scharfe Kanten an der konvexen Artikulationsfläche 5 vermieden. Dies im Unterschied zu aus dem Stand der Technik bekannten nutförmigen Einkerbungen des Kugelgelenkkopfs. In Bezug auf ihre jeweilige Haupterstreckungsrichtung sind die Abflachungsflächen 7a bis 7l entsprechend der kugelförmigen Kontur des Kugelgelenkkopfs 2 bzw. der modifizierten Kugelfläche K gekrümmt längserstreckt. Anhand Fig. 6 ist zudem ersichtlich, dass der Kugelgelenkkopf 2 vorliegend mit einem Radius R ausgeführt ist. Dabei weisen die Abflachungsflächen 7a bis 7l - jedenfalls in Bezug auf die gezeigte Schnittebene - keinerlei Radius und somit weder eine konvexe noch eine konkave Krümmung auf.

Bei der gezeigten Ausführungsform weist die modifizierte Kugelfläche K im Vergleich zu der nicht-modifizierten Kugelfläche K' des nicht-abgeflachten und insoweit üblichen Kugelgelenkkopfs 2' (Fig. 3) eine um 40 % reduzierte Oberfläche zur gleitbeweglichen Artikulation mit der konkaven Artikulationsfläche 6 auf. Mit anderen Worten ausgedrückt, findet zwischen den Abflachungsflächen 7a bis 7l und der konkaven Artikulationsfläche 6 kein gleitbeweglicher Kontakt statt, wodurch die wirksame Kontaktfläche des Kugelgelenkkopfs 2 entsprechend reduziert ist.

Bei zeichnerisch nicht dargestellten Ausführungsformen ist die modifizierte Kugelfläche vergleichsweise um 25 % bis 50 % gegenüber einer im Durchmesser identischen, nicht-abgeflachten Kugelfläche reduziert.

Vorliegend sind die Abflachungsflächen 7a bis 7l in Umfangsrichtung U des Kugelgelenkkopfs 2 gleichmäßig beabstandet angeordnet (Fig. 4, 5, 6). Bei einer zeichnerisch nicht dargestellten Ausführungsform ist stattdessen eine ungleichmäßige Beabstandung vorgesehen.

Zudem sind die Abflachungsflächen 7a bis 7l vorliegend jeweils ausgehend von einem unterseitigen Schaftbereich 21 des Kugelgelenkkopfs 2 bis in einen oberseitigen Polbereich 22 des Kugelgelenkkopfs 2 durchgängig längserstreckt. Hierdurch bilden die Abflachungsflächen 7a bis 7l im Zusammenwirken mit der konkaven Artikulationsfläche 6 jeweils einen durchgängig längserstreckten Kapillarspalt S aus (Fig. 1). Dabei ist anhand Fig. 1 aus Gründen der Übersichtlichkeit lediglich einer der mehreren Kapillarspalte mittels des dortigen Bezugszeichens "S" gekennzeichnet. Es versteht sich, dass entsprechend der Anzahl der Abflachungsflächen 7a bis 7l mehrere in Umfangsrichtung des Kugelgelenkkopfs 2 benachbart angeordnete Kapillarspalte ausgebildet sind. Entlang der Kapillarspalte findet im implantierten Zustand der Kugelgelenkendoprothese 1 ein kapillarer Transport von Gelenkflüssigkeit statt.

Hierdurch kann eine verbesserte Schmierung der gleitbeweglichen Artikulation zwischen dem Kugelgelenkkopf 2 und der Kugelgelenkpfanne 3 erreicht werden. Dies vermindert dem reibungsbedingten Abrieb und damit den Verschleiß der Kugelgelenkendoprothese 1.

Der kapillare Transport der Gelenkflüssigkeit wird durch eine entsprechende Dimensionierung der Abflachung der Abflachungsflächen 7a bis 7l ermöglicht. Um eine ausreichende kapillare Steighöhe der Gelenkflüssigkeit innerhalb der Kapillarspalte zu gewährleisten, sind die Abflachungsflächen 7a bis 7l bei der gezeigten Ausführungsform jeweils um 0,1 mm gegenüber der gedachten nicht-modifizierten Kugelfläche K' abgeflacht. Hierdurch wird ein hinreichend geringes Spaltmaß der Kapillarspalte erreicht. Bei einer zu starken, über wenige Zehntel Millimeter hinausgehenden Abflachung wird stattdessen ein Spaltmaß erreicht, das keinen oder jedenfalls keinen ausreichenden Kapillareffekt bewirkt.

Bei zeichnerisch nicht dargestellten Ausführungsformen bewegt sich die Abflachung der Abflachungsfläche maßlich zwischen 0,03 mm und 0,3 mm.

Vorliegend beziehen sich die vorstehenden maßlichen Angaben auf eine maximale Abflachung der Abflachungsflächen 7a bis 7l. Die maximale Abflachung ist bei der gezeigten Ausführungsform einends, an einem an den Schaftbereich 21 angrenzenden Stirnendbereich der jeweiligen Abflachungsfläche 7a bis 7l vorgesehen. Ausgehend von dort nimmt die Abflachung der Abflachungsflächen 7a bis 7l in Polrichtung P (Fig. 6) des Kugelgelenkkopfs 2 kontinuierlich ab und geht im Polbereich 22 des Kugelgelenkkopfs 2 in die modifizierte Kugelfläche K über. Dies ist deutlich anhand Fig. 5 ersichtlich. Mit anderen Worten ausgedrückt, verringert sich die Abflachung der Abflachungsflächen 7a bis 7l ausgehend von dem Schaftbereich 21 entlang der jeweiligen Haupterstreckungsrichtung und geht im Polbereich 22 gegen null. Infolge der sich kontinuierlich verringernden Abflachung weisen die Abflachungsflächen 7a bis 7l entlang ihrer jeweiligen Haupterstreckungsrichtung eine veränderliche Breite auf. Diese ist im Schaftbereich 21 maximal und im Polbereich 22 minimal. Dabei geht die besagte Breite im Polbereich 22 jeweils gegen null.

Bei der gezeigten Ausführungsform sind die Abflachungsflächen 7a bis 7l in Bezug auf die Polrichtung P gerade längserstreckt (Fig. 6).

Bei zeichnerisch nicht dargestellten Ausführungsformen ist dementgegen eine winklige oder wendelförmige Längserstreckung der Abflachungsflächen vorgesehen.

Vorliegend weist der Kugelgelenkkopf 2 insgesamt zwölf Abflachungsflächen 7a bis 7l auf. Diese sind um jeweils 30° in Umfangsrichtung des Kugelgelenkkopfs 2 zueinander versetzt angeordnet.

Bei zeichnerisch dargestellten Ausführungsformen weist der Kugelgelenkkopf 2 zwischen zwei und 20 Abflachungsflächen auf.

Die Abflachungsflächen 7a bis 7l können mittels abrasiver oder nicht-abrasiver Fertigungsverfahren an dem Kugelgelenkkopf 2 ausgebildet werden. Hierzu hat sich ein abschnittsweises Schleifen, insbesondere ein Planschleifen, des Kugelgelenkkopfs 2 als besonders vorteilhaft erwiesen.

## Patentansprüche

1. Kugelgelenkkopf (2) mit einer konvexen Artikulationsfläche (5), die zur kugelgelenkigen gleitbeweglichen Artikulation mit einer konkaven Artikulationsfläche (6) einer Kugelgelenkpfanne (3) einer der Kugelgelenkendoprothese (1) vorgesehen ist, wobei die konvexe Artikulationsfläche (5) mehrere benachbart angeordnete und radial abgeflachte Abflachungsflächen (7a bis 7l) aufweist, wodurch die konvexe Artikulationsfläche (5) in Form einer modifizierten Kugelfläche (K) ausgebildet ist, die - im Vergleich zu einer gedachten nicht-modifizierten Kugelfläche (K') - eine um die Abflachungsflächen (7a bis 7l) reduzierte Oberfläche zur Artikulation mit der konkaven Artikulationsfläche (6) aufweist, **dadurch gekennzeichnet, dass** die modifizierte Kugelfläche (K) im Vergleich zu der gedachten nicht-modifizierten Kugelfläche (K') eine um 25 % bis 50 %, bevorzugt um 40 %, reduzierte Oberfläche zur Artikulation mit der konkaven Artikulationsfläche (6) aufweist, wobei
die Abflachungsflächen (7a bis 7l) jeweils in einer ersten Erstreckungsrichtung plan erstreckt sind und in einer senkrecht zu der ersten Erstreckungsrichtung orientierten zweiten Erstreckungsrichtung gekrümmt erstreckt sind.

2. Kugelgelenkendoprothese (1), insbesondere Hüftendoprothese, mit einem Kugelgelenkkopf (2) nach Anspruch 1 und mit einer Kugelgelenkpfanne (3), die eine konkave Artikulationsfläche (6) aufweist, wobei die konvexe Artikulationsfläche (5) und die konkave Artikulationsfläche (6) kugelgelenkig artikulierend gleitbeweglich zusammenwirken.

3. Kugelgelenkendoprothese (1) nach Anspruch 2, wobei die Abflachungsflächen (7a bis 7l) in Umfangsrichtung (U) des Kugelgelenkkopfs (2) gleichmäßig beabstandet angeordnet sind.

4. Kugelgelenkendoprothese (1) nach einem der Ansprüche 2-3, wobei die Abflachungsflächen (7a bis 7l) jeweils ausgehend von einem unterseitigen Schaftbereich (21) des Kugelgelenkkopfs (2) bis in einen oberseitigen Polbereich (22) des Kugelgelenkkopfs (2) durchgängig längserstreckt sind, wodurch jeweils ein durchgängig längserstreckter Kapillarspalt (S) zum kapillaren Transport von Gelenkflüssigkeit ausgebildet ist.

5. Kugelgelenkendoprothese (1) nach einem der Ansprüche 2-4, wobei die Abflachungsflächen (7a bis 7l) um jeweils 0,03 mm bis 0,3 mm, bevorzugt um 0,1 mm, gegenüber der gedachten nicht-modifizierten Kugelfläche (K') abgeflacht sind.

6. Kugelgelenkendoprothese (1) nach einem der Ansprüche 2-5, wobei die Abflachungsflächen (7a bis 7l) in Polrichtung (P) des Kugelgelenkkopfs (2) jeweils abnehmend abgeflacht sind und im Polbereich (22) des Kugelgelenkkopfs (2) stetig in die modifizierte Kugelfläche (K) übergehen.

7. Kugelgelenkendoprothese (1) nach einem der Ansprüche 2-6, wobei die Abflachungsflächen (7a bis 7l) in Bezug auf die Polrichtung (P) des Kugelgelenkkopfs (2) jeweils gerade oder winklig oder wendelförmig längserstreckt sind.

8. Kugelgelenkendoprothese (1) nach einem der Ansprüche 2-7, wobei zwischen zwei und 20, bevorzugt zwölf, Abflachungsflächen (7a bis 7l) vorgesehen sind.

## Claims

1. Ball joint head (2) having a convex articulation surface (5), which is provided for spherical sliding articulation with a concave articulation surface (6) of a ball joint socket (3) of a ball joint endoprosthesis (1), wherein the convex articulation surface (5) has a plurality of adjacently arranged and radially flattened flat surfaces (7a to 71), as a result of which the convex articulation surface (5) is designed in the form of a modified spherical surface (K), which - compared to an imaginary non-modified spherical surface (K') - has a surface, reduced by the flat surfaces (7a to 71), for articulation with the concave articulation surface (6),
**characterized in that**
the modified spherical surface (K) has, compared to the imaginary non-modified spherical surface (K'), a surface reduced by 25% to 50%, preferably by 40%, for articulation with the concave articulation surface (6), wherein the flat surfaces (7a to 71) each extend in a planar manner in a first direction of extent and extend in a curved manner in a second direction of extent oriented perpendicular to the first direction of extent.

2. Ball joint endoprosthesis (1), in particular a hip endoprosthesis, having a ball joint head (2) according to Claim 1 and having a ball joint socket (3), which has a concave articulation surface (6), wherein the convex articulation surface (5) and the concave articulation surface (6) interact with spherical sliding articulation.

3. Ball joint endoprosthesis (1) according to Claim 2, wherein the flat surfaces (7a to 71) are arranged uniformly spaced apart in the circumferential direction (U) of the ball joint head (2).

4. Ball joint endoprosthesis (1) according to either of Claims 2-3, wherein the flat surfaces (7a to 71) each extend longitudinally continuously from an underside shaft region (21) of the ball joint head (2) as far as an upper pole region (22) of the ball joint head (2), as a result of which in each case a continuously elongate capillary gap (S) is formed for the capillary transport of synovial fluid.

5. Ball joint endoprosthesis (1) according to any one of Claims 2-4, wherein the flat surfaces (7a to 71) are flattened in each case by 0.03 mm to 0.3 mm, preferably by 0.1 mm, compared to the imaginary non-modified spherical surface (K').

6. Ball joint endoprosthesis (1) according to any one of Claims 2-5, wherein the flat surfaces (7a to 71) are each flattened decreasingly in the pole direction (P) of the ball joint head (2) and transition continuously into the modified spherical surface (K) in the pole region (22) of the ball joint head (2).

7. Ball joint endoprosthesis (1) according to any one of Claims 2-6, wherein the flat surfaces (7a to 71) each extend longitudinally straight or at an angle or helically with respect to the pole direction (P) of the ball joint head (2).

8. Ball joint endoprosthesis (1) according to any one of Claims 2-7, wherein between two and 20, preferably twelve, flat surfaces (7a to 71) are provided.

## Revendications

1. Tête de joint sphérique (2) avec une surface d'articulation convexe (5), qui est prévue pour l'articulation à joint sphérique avec mouvement de glissement avec une surface d'articulation concave (6) d'une cupule de joint sphérique (3) d'une endoprothèse à joint sphérique (1), la surface d'articulation convexe (5) présentant plusieurs surfaces d'aplatissement (7a à 71) agencées de manière voisine et aplaties radialement, moyennant quoi la surface d'articulation convexe (5) est réalisée sous la forme d'une surface sphérique modifiée (K) qui - en comparaison d'une surface sphérique imaginaire non modifiée (K') - présente une surface réduite par les surfaces d'aplatissement (7a à 71) pour l'articulation avec la surface d'articulation concave (6), **caractérisée en ce que**
la surface sphérique modifiée (K) présente, en comparaison de la surface sphérique imaginaire non modifiée (K'), une surface réduite de 25 % à 50 %, de préférence de 40 %, pour l'articulation avec la surface d'articulation concave (6), les surfaces d'aplatissement (7a à 71) s'étendant chacune dans une première direction d'extension de manière plane et s'étendant de manière incurvée dans une deuxième direction d'extension orientée perpendiculairement à la première direction d'extension.

2. Endoprothèse à joint sphérique (1), notamment endoprothèse de hanche, avec une tête de joint sphérique (2) selon la revendication 1 et avec une cupule de joint sphérique (3) qui présente une surface d'articulation concave (6), la surface d'articulation convexe (5) et la surface d'articulation concave (6) coopérant avec mouvement de glissement en s'articulant à joint sphérique.

3. Endoprothèse à joint sphérique (1) selon la revendication 2, dans laquelle les surfaces d'aplatissement (7a à 71) sont agencées espacées de manière uniforme dans la direction circonférentielle (U) de la tête de joint sphérique (2).

4. Endoprothèse à joint sphérique (1) selon l'une quelconque des revendications 2 à 3, dans laquelle les surfaces d'aplatissement (7a à 71) sont respectivement étendues longitudinalement de manière continue à partir d'une zone de tige (21) côté inférieur de la tête de joint sphérique (2) jusque dans une zone polaire (22) côté supérieur de la tête de joint sphérique (2), moyennant quoi une fente capillaire (S) étendue longitudinalement de manière continue pour le transport capillaire de liquide de joint est réalisée respectivement.

5. Endoprothèse à joint sphérique (1) selon l'une quelconque des revendications 2 à 4, dans laquelle les surfaces d'aplatissement (7a à 71) sont aplaties chacune de 0,03 mm à 0,3 mm, de préférence de 0,1 mm, par rapport à la surface sphérique imaginaire non modifiée (K').

6. Endoprothèse à joint sphérique (1) selon l'une quelconque des revendications 2 à 5, dans laquelle les surfaces d'aplatissement (7a à 71) sont respectivement aplaties de manière décroissante dans la direction polaire (P) de la tête de joint sphérique (2) et se transforment de manière continue en la surface sphérique modifiée (K) dans la zone polaire (22) de la tête de joint sphérique (2).

7. Endoprothèse à joint sphérique (1) selon l'une quelconque des revendications 2 à 6, dans laquelle les surfaces d'aplatissement (7a à 71) sont respectivement allongées longitudinalement de manière rectiligne ou angulaire ou hélicoïdale par rapport à la direction polaire (P) de la tête de joint sphérique (2).

8. Endoprothèse à joint sphérique (1) selon l'une quelconque des revendications 2 à 7, dans laquelle il est prévu entre deux et 20, de préférence douze, surfaces d'aplatissement (7a à 71).
